# EUROPEAN PATENT APPLICATION

(11) **EP 3 225 242 A1**
(43) Date of publication of application: **04.10.2017**
(21) Application number: 15862902.2
(22) Date of filing: 27.11.2015
(51) Int. Cl.: A61K 31/575, A61K 31/704, A61P 1/02, A61P 1/04, A61P 9/04, A61P 9/10, A61P 11/00, A61P 17/00, A61P 19/04, A61P 19/10, A61P 29/00, A61P 35/04

(54) **MATRIX METALLOPROTEINASE PRODUCTION INHIBITOR**

(30) Priority: 28.11.2014 JP 2014241923
(71) Applicant: Morinaga Milk Industry Co., Ltd., Minato-ku Tokyo 108-8384 (JP)
(72) Inventor: SAITO, Marie, Zama-shi Kanagawa 252-8583 (JP); MISAWA, Eriko, Zama-shi Kanagawa 252-8583 (JP); TANAKA, Miyuki, Zama-shi Kanagawa 252-8583 (JP); YAO, Ruiqing, Zama-shi Kanagawa 252-8583 (JP)
(74) Representative: Vossius & Partner Patentanwälte Rechtsanwälte mbB
(86) International application number: PCT/JP2015/083476
(87) International publication number: WO 2016/084957

(57) **Abstract**

Provided is a matrix metalloproteinase production inhibitor which can be daily taken safely. In the matrix metalloproteinase production inhibitor, a compound selected from the group consisting of a lophenol compound and a cyclolanostane compound is used as an active ingredient.

## Description

### TECHNICAL FIELD

The present invention relates to a matrix metalloproteinase production inhibitor.

### BACKGROUND ART

An extracellular matrix (ECM) is a structure which is formed at the periphery of animal cells, and not only fills extracellular gaps, but also plays a role as a scaffold for fixing and adhering each cell. Additionally, the extracellular matrix is also known to be involved in maintenance of homeostasis of a living body, and is also known to regulate proliferation, differentiation and migration of cells, cell death and the like. As the main extracellular matrix, collagen, fibronectin, proteoglycan, laminin and the like are known.

As an enzyme which degrades these extracellular matrices, there are matrix metalloproteinases (hereinafter, referred to as MMP). MMPs are metal-demanding neutral proteases which are common in the structure and the function, and function extracellularly and on cell membranes. MMP is composed of several domain structures which are structurally conserved well, and has a signal sequence, an active center region, and a propeptide region being a region for controlling it, as the basic structure (Non-Patent Document 1, Non-Patent Document 2).

Among MMPs, MMP-1, MMP-8, MMP-13 and MMP-18 belong to a collagenase group, and degrade type I, II and III collagens, among extracellular matrices. MMP-2 and MMP-9 belong to a gelatinase group, and are known to degrade type I, II and III collagens, additionally, further degrade collagen which was degraded by collagenase or the like (denatured collagen), and be also involved in degradation of type IV collagen being the main component of a basement membrane and degradation of elastin. MMP-3, 10, and 11 belong to a stromelysin group, and degrade matrix proteins such as laminin, fibronectin, proteoglycan and the like. Additionally, there are MMP-12 being a metalloelastase with the elastin degrading activity having the structure similar thereto, and MMP-7 and MMP-26 belonging to a matrilysin group. MMP-14, 15 and 16 belonging to a membrane-type MMP (MMT-MP) group, and MMP-17, 24 and 25 have a transmembrane region, and it is known that all are involved in degradation of an extracellular matrix (Non-Patent Document 1, Non-Patent Document 2).

The function of MMP which degrades extracellular matrix components is very strictly controlled in a living body, and is controlled in multiple stages such as 1) gene expression, 2) conversion of a precursor into an active form and, 3) binding of an inhibiting factor to active form MMP (Non-Patent Document 1). MMP works in generation of embryos, differentiation of tissues and neovascularization, by destructing an extracellular matrix, and additionally, is also known to be involved in infiltration and metastasis of cancers, arthritis and periodontal diseases, ulceration of tissues (corneal ulcer, gastric ulcer, or epidermal ulcer) (Non-Patent Document 1, Non-Patent Document 2).

As a drug having an inhibitory effect on MMP, CGS27023a (Non-Patent Document 3), collagen tripeptide (Non-Patent Document 4), a flavonoid compound (Patent Document 1), a catechin compound (Patent Document 2) and the like are known. Additionally, as one having an effect of inhibiting production of MMP, a winged bean extract (Patent Document 3), menatetrenone (Patent Document 4) and the like are known.

Meanwhile, it has been found out that, among phytosterols, a compound having a cyclolanostane skeleton and a compound having a lophenol skeleton have an action of reducing the amount of the lipid peroxide in blood in an arteriosclerosis model animal, and have an action of suppressing the number of the plaque formation of thoracic aorta, and use as an antioxidant has been proposed (Patent Document 5). However, a relationship of these compounds with MMP has not been known yet.

### PRIOR ART DOCUMENTS

### NON-PATENT DOCUMENTS

Non-Patent Document 1: Chemistry and Organisms, Vol. 35, No. 12, p. 816-818, 1997
Non-Patent Document 2: Biological Physicochemistry, Vol. 47, p. 111-116, 2003
Non-Patent Document 3 : Journal of Medicinal Chemistry, Vol. 40, p. 2525-2532, 1997
Non-Patent Document 4: Prev Nutr Food Sci, Vol. 17, p. 245-253, 2012

### PATENT DOCUMENTS

Patent Document 1: JP-A No. 8-104628
Patent Document 2: JP-A No. 2000-226329
Patent Document 3: JP-A No. 2012-206984
Patent Document 4: WO 2004/093858
Patent Document 5: WO 2010/058795

### DISCLOSURE OF THE INVENTION

### PROBLEMS TO BE SOLVED BY THE INVENTION

As described in Patent Documents 1 to 4, materials exhibiting the MMP inhibitory action have been proposed, and development of a material which can be safely ingested has been further demanded.

Then, an object of the present invention is to provide a functional material being able to inhibit MMP production, which can be routinely ingested safely, and a medicine utilizing this.

### MEANS TO SOLVE THE PROBLEMS

The first invention for solving the above-described problem is a matrix metalloproteinase production inhibitor comprising a compound selected from the group consisting of a lophenol compound and a cyclolanostane compound, as an active ingredient (hereinafter, referred to as "inhibitor of the present invention").

In a preferable embodiment of the present invention, the above-described inhibitor comprises the above-described compound at a total amount of 0.00001% by mass or more.

In a preferable embodiment of the present invention, the above-described compound is selected from the group consisting of 4-methylcholest-7-en-3-ol, 4-methylergost-7-en-3-ol, 4-methylstigmast-7-en-3-ol, 9,19-cyclolanostan-3-ol and 24-methylene-9,19-cyclolanostan-3-ol.

The inhibitor of the present invention is effective in inhibiting production of, particularly, a matrix metalloproteinase selected from the group consisting of matrix metalloproteinase-2, matrix metalloproteinase-9, matrix metalloproteinase-12 and matrix metalloproteinase-13.

Further, the second invention for solving the above-described problem is use of a compound selected from the group consisting of a lophenol compound and a cyclolanostane compound in the manufacture of a matrix metalloproteinase production inhibitor, and a preferable embodiment of the compound is as described above.

Further, the second invention includes the following embodiment:
Use of a composition comprising a compound selected from the group consisting of a lophenol compound and a cyclolanostane compound at a total amount of 0.00001% by mass or more in the manufacture of a matrix metalloproteinase production inhibitor.

In a preferable embodiment of the present invention, the matrix metalloproteinase production inhibitor is an inhibitor of production of a matrix metalloproteinase selected from the group consisting of matrix metalloproteinase-2, matrix metalloproteinase-9, matrix metalloproteinase-12 and matrix metalloproteinase-13.

The second invention includes use of a compound selected from the group consisting of a lophenol compound and a cyclolanostane compound in the manufacture of a medicine for preventing or improving symptoms due to degradation of an extracellular matrix.

Further, the embodiment includes also the following:
Use of a composition comprising a compound selected from the group consisting of a lophenol compound and a cyclolanostane compound at a total amount of 0.00001% by mass or more in the manufacture of a medicine for preventing or improving symptoms due to degradation of an extracellular matrix.

Further, the third invention for solving the above-described problem is a compound selected from the group consisting of a lophenol compound and a cyclolanostane compound, for use in inhibiting matrix metalloproteinase production, and a preferable embodiment of the compound is as described above.

In a preferable embodiment of the present invention, the above-described compound is used for inhibiting production of a matrix metalloproteinase selected from the group consisting of matrix metalloproteinase-2, matrix metalloproteinase-9, matrix metalloproteinase-12 and matrix metalloproteinase-13.

Further, it is preferable that the above-described compound is used for preventing or improving symptoms due to degradation of an extracellular matrix.

Further, the fourth invention for solving the above-described problem is a composition comprising a compound selected from the group consisting of a lophenol compound and a cyclolanostane compound at a total amount of 0.00001% by mass or more for use in inhibiting matrix metalloproteinase production, and a preferable embodiment of the compound is as described above.

In the fourth invention, the above-described composition is preferably food or drink.

In a preferable embodiment of the present invention, the above-described composition is used for inhibiting production of a matrix metalloproteinase selected from the group consisting of matrix metalloproteinase-2, matrix metalloproteinase-9, matrix metalloproteinase-12 and matrix metalloproteinase-13.

Further, it is preferable that the above-described composition is used for preventing or improving symptoms due to degradation of an extracellular matrix.

Further, the fifth invention for solving the above-described problem is a method for inhibiting production of a matrix metalloproteinase, comprising administering a compound selected from the group consisting of a lophenol compound and a cyclolanostane compound to a subject in need of inhibition of production of a matrix metalloproteinase, and a preferable embodiment of the compound is as described above.

In a preferable embodiment of the present invention, the matrix metalloproteinase is selected from the group consisting of matrix metalloproteinase-2, matrix metalloproteinase-9, matrix metalloproteinase-12 and matrix metalloproteinase-13.

Further, the embodiment includes the following:
A method for improving or preventing a disease or a symptom due to degradation of an extracellular matrix, comprising administering a compound selected from the group consisting of a lophenol compound and a cyclolanostane compound to a subject having a disease or a symptom due to degradation of an extracellular matrix.

Further, in the fifth invention, the following is a preferable embodiment.

Administration of a composition comprising a compound selected from the group consisting of a lophenol compound and a cyclolanostane compound at a total amount of 0.00001% by mass or more.

The disease or the symptom due to degradation of an extracellular matrix includes an inflammatory disease. The disease or the symptom also includes gingivitis or periodontitis, tissue ulcer, skin disease, arthritis, infiltration or metastasis of cancers, and endometriosis, and the above-described compound or the above-described composition comprising this is particularly effective in preventing or improving these symptoms.

### EFFECT OF THE INVENTION

The inhibitor of the present invention can be safely ingested, and effectively inhibits production of a matrix metalloproteinase. The inhibitor of the present invention is useful for preventing, improving or treating arthritis, inflammatory disease, periodontal disease, bone disease, infiltration and metastasis of cancers, aneurysm disease, dyskeratosis, skin inflammatory diseases due to ultraviolet or the like, skin aging and the like.

### BRIEF DESCRIPTION OF THE DRAWINGS

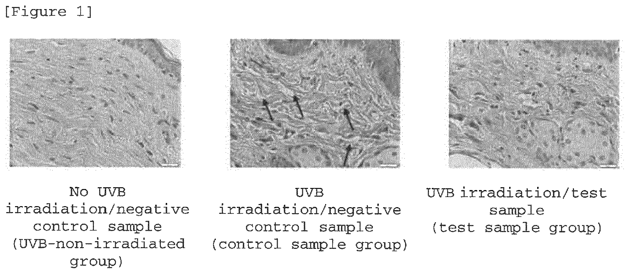

Fig. 1 is an optical micrograph of an extracellular matrix tissue section of a mouse of each group in Example 1. In the figure, an arrow indicates places where fracture of fibers is observed.

### DISCRIPTION OF EMBODIMENTS

Then, a preferable embodiment of the present invention will be illustrated in detail. The present invention is not limited to the following preferable embodiments, and can be freely modified in the scope of the present invention. In addition, percentage in the present description is indication by mass, unless otherwise indicated.

The inhibitor of the present invention contains, as an active ingredient, a compound selected from the group consisting of lophenol compounds (Compound 1) and cyclolanostane compounds (Compound 2). Compound 1 and Compound 2 are represented by the following general formula (1) and the general formula (2), respectively.

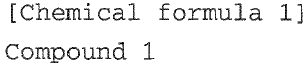

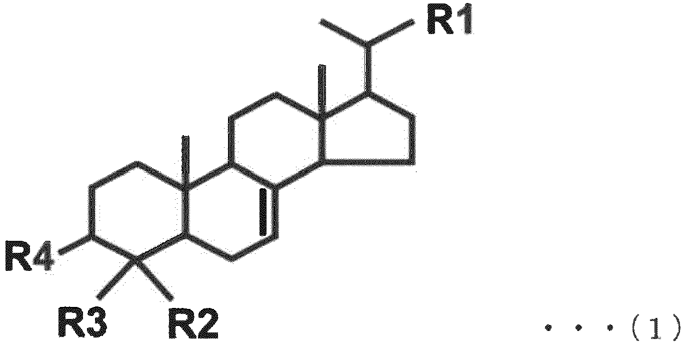

In the general formula (1), R1 is an alkyl group or an alkenyl group including one or two double bonds, which is straight or branched chain having 5 to 16 carbon atoms. The alkyl or alkenyl group may be a substituted alkyl or alkenyl group, in which one or two hydrogen atoms are substituted with a hydroxyl group and/or a carbonyl group.

R2 and R3 each are independently a hydrogen atom or an alkyl group having 1 to 3 carbon atoms. Herein, as the alkyl group having 1 to 3 carbon atoms, a methyl group, an ethyl group and the like are preferable, and a methyl group is particularly preferable. The alkyl group may be a substituted alkyl group in which at least one hydrogen atom is substituted with a hydroxyl group and/or a carbonyl group.

[Chemical formula 2] -CH₂-OH -CH₂-COOH -CH₂-CH₂-OH -CH₂-CH₂-COOH -CH(OH)-CH₃ -CH(COOH)-CH₃

R4 forms C=O with a carbon atom constituting the ring, or is -OH or -OCOCH₃.

In the general formula (1), R1 is preferably any of groups represented by the following formulae.

[Chemical formula 3] -CH₂-CH₂-CH(CH₂-CH₃)-CH(CH₃)₂ -CH₂-CH₂-CH=C(CH₃)₂ -CH₂-CH=C(CH₃)-CH(CH₃)₂ -CH₂-CH₂-C(=CH-CH₃)-CH(CH₃)₂ -CH₂-CH₂-CH(Ra)=C(CH₃)Rb

(wherein Ra and Rb are any of a hydrogen atom, a hydroxyl group and a methyl group)

-CH₂-CH₂-CH(Rc)-CH(CH₃)Rd

(wherein Rc and Rd are any of a hydrogen atom, a hydroxyl group and a methyl group)

In the general formula (1), it is preferable that one of R2 and R3 is a hydrogen atom, and the other is a methyl group, and it is preferable that R4 is a hydroxy group.

Compound 1 includes preferably 4-methylcholest-7-en-3-ol, 4-methylergost-7-en-3-ol and 4-methylstigmast-7-en-3-ol. Respective compounds have structures represented by the following formulae, respectively.

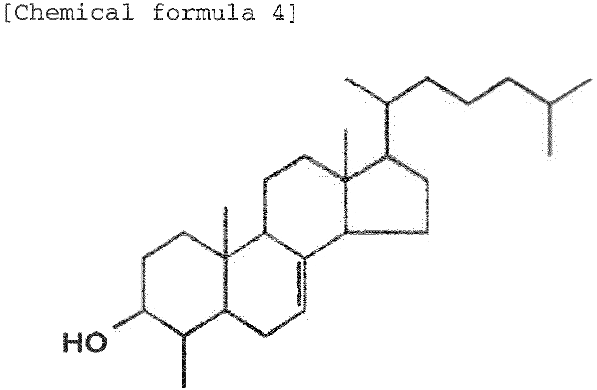

### 4-Methylcholest-7-en-3-ol

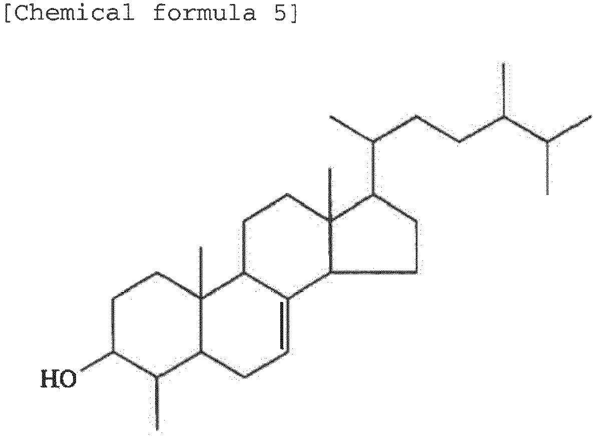

### 4-Methylergost-7-en-3-ol

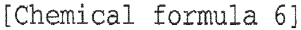

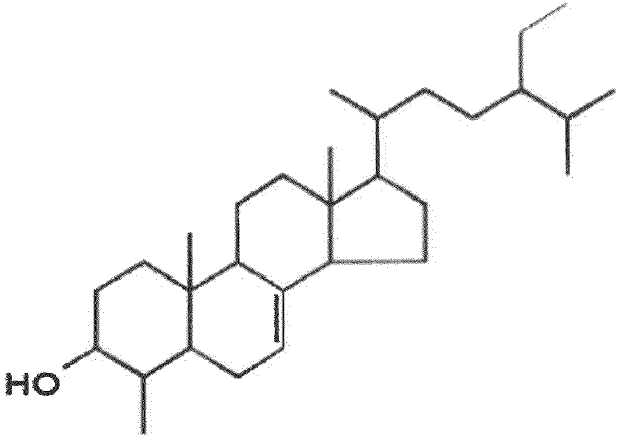

### 4-Methylstigmast-7-en-3-ol

Compound 1 can be chemically manufactured in accordance with the known manufacturing processes.

Compound 1 can be synthesized, for example, in accordance with supplement data described in Vitali Matyash et al., PLOS BIOLOGY, Volume 2, Issue 10, e280, 2004.

Further, it is known that Compound 1 is contained in plants, and Compound 1 can be manufactured in accordance with the known process for manufacturing lophenol (Biochemistry Experimental Method 24, Fat Lipid Metabolism Experimental Method, authored by Akihiro YAMADA, Gakkai Shuppan Center, p. 174, 1989).

For example, Compound 1 can be extracted from plants which are known to contain Compound 1, using a method such as a hot water extraction method, an organic solvent extraction method, a supercritical extraction method and a subcritical extraction method (see, e.g., Japanese Patent No. 3905913). Compound 1 can be extracted, for example, from plants belonging to family *Liliaceae,* family *Leguminosae,* family *Gramineae,* family *Solanaceae* and family *Musaseae.*

The molecular weight and the structure of Compound 1 manufactured as described above can be determined or confirmed by a mass spectrometry (MS), a nuclear magnetic resonance spectral (NMR) method.

Further, Compound 1 may be a pharmaceutically acceptable salt. The pharmaceutically acceptable salt includes both metal salts (inorganic salts) and organic salts, and as a list of them, that described in "Remington's Pharmaceutical Sciences, 17th edition, 1985, p. 1418" is exemplified.

Specifically, inorganic salts such as a hydrochloride, a sulfate, a phosphate, a diphosphate, and a hydrobromide, and organic salts such as a malate, a maleate, a fumarate, a tartrate, a succinate, a citrate, an acetate, a lactate, a methanesulfonate, a p-toluenesulfonate, a pamoate, a salicylate, and a stearate are included without limitation.

Meanwhile, Compound 1 may be a salt with a metal such as sodium, potassium, calcium, magnesium and aluminum, or a salt with an amino acid such as lysine. Moreover, there may also be used a solvate such as a hydrate of the compounds or pharmaceutically acceptable salts thereof.

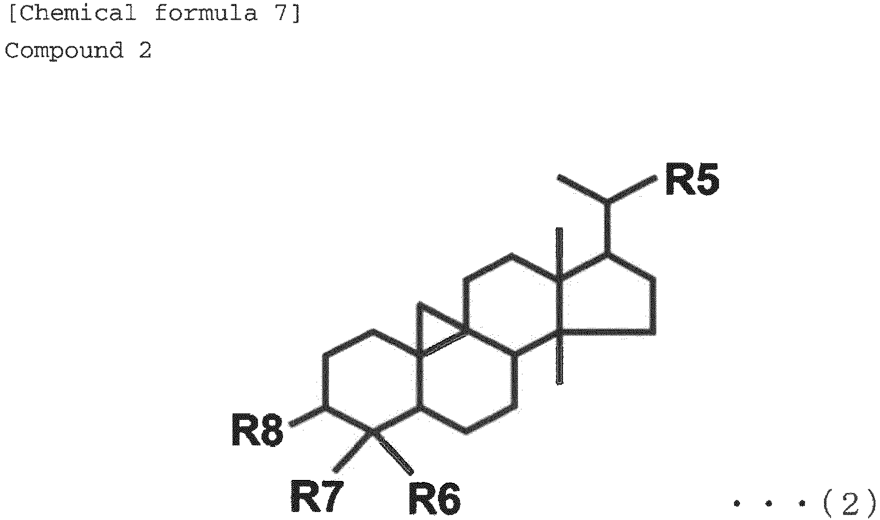

In the general formula (2), R5 is an alkyl group or an alkenyl group including one or two double bonds, which is straight or branched chain having 6 to 8 carbon atoms. The alkyl or alkenyl group may be a substituted alkyl or alkenyl group in which one or two hydrogen atoms are substituted with a hydroxyl group and/or a carbonyl group.

R6 and R7 each are independently a hydrogen atom or a methyl group. R8 forms C=O with a carbon atom constituting the ring, or is any of the following formulae.

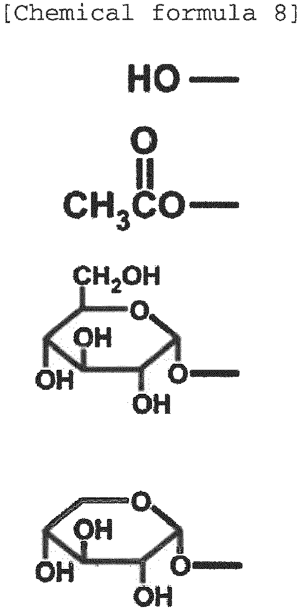

In the general formula (2), R5 is preferably any of groups represented by the following formulae.

[Chemical formula 9] -CH₂-CH₂-CH₂-CH(CH₃)₂ -CH₂-CH₂-CHRe-C(CH₃)₂Rf

(Re is a hydrogen atom, a hydroxyl group or a methyl group, and Rf is a hydrogen atom or a hydroxyl group)

-CH₂-CH₂-CH(CH₂-CH₃)-CH(CH₃)₂

-CH₂-CH₂-CHRg-C(CH₃)=CH₂

(Rg is a hydrogen atom, a hydroxyl group or a methyl group)

-CH₂-CH₂-C(=O)-CH(CH₃)=CH₂

-CH₂-CH₂-C(=CH₂)-CH(CH₃)₄

-CH₂-CH₂-CH=C(CH₃)₂

-CH₂-CH₂=C(CH₃)-CH(CH₃)₂

-CH₂-CH₂-C(=CH-CH₃)-CH(CH₃)₂

Further, in the general formula (2), it is preferable that one of R6 and R7 is a hydrogen atom, and the other is a methyl group, and it is preferable that R8 is a hydroxy group.

Compound 2 includes preferably 9,19-cyclolanostan-3-ol and 24-methylene-9,19-cyclolanostan-3-ol. Respective compounds have structures represented by the following formulae, respectively.

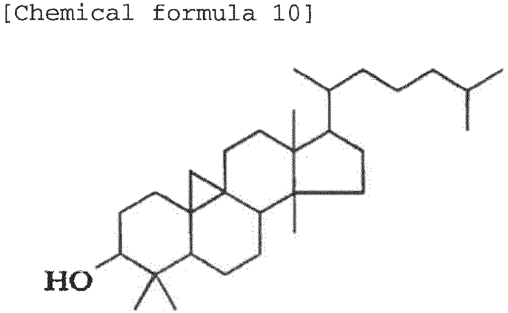

### 9,19-Cyclolanostan-3-ol

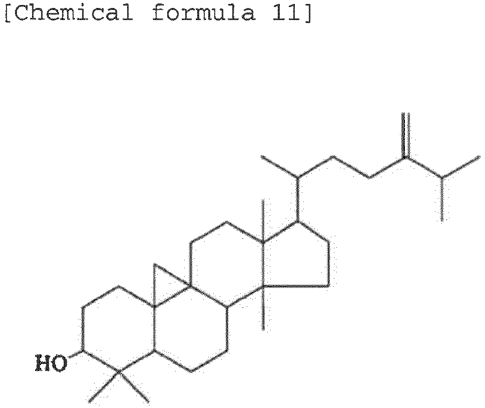

### 24-Methylene-9,19-cyclolanostan-3-ol

Compound 2 can be chemically manufactured in accordance with the known manufacturing processes. For example, 24-methylene-9,19-cyclolanostan-3-ol (trivial name: 24-methylenecycloartanol) can be manufactured by the methods disclosed in JP-A No. 57-018617 and WO 2012/023599 (method of synthesis from γ-oryzanol). Alternatively, Compound 2 can be manufactured using a hydrolysate of cycloartenol ferulate as a starting substance, by the method disclosed in JP-A No. 2003-277269.

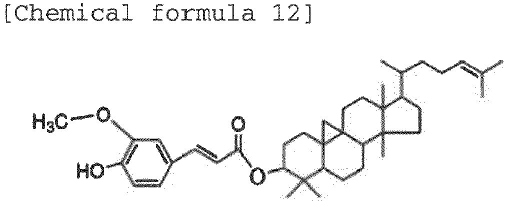

### Cycloartenol ferulate

Further, Compound 2 is also known to be contained in a plant belonging to family *Liliaceae,* family *Leguminosae,* family *Gramineae,* family *Solanaceae,* or family *Musaseae* (see [Phytochemistry, USA, 1977, vol. 16, pp. 140-141], [Handbook of phytochemical constituents of GRAS herbs and other economic plants, 1992, USA, CRC Press] or [Hager's Handbuch der Pharmazeutischen Praxis, vol. 2-6, 1969-1979, Deutschland, Springer Verlag, Berlin]). Hence, Compound 2 can be extracted from these plants using the known methods such as an organic solvent extraction method or a hot water extraction method (see, e.g., Japanese Patent No. 3924310).

The molecular weight and the structure of the compound manufactured as described above can be determined or confirmed, for example, by mass spectrometry (MS) and nuclear magnetic resonance spectrometry (NMR).

Further, Compound 2 may be a pharmaceutically acceptable salt. Such a salt is as exemplified concerning Compound 1.

The inhibitor of the present invention contains, as an active ingredient, a compound selected from the group consisting of Compound 1 and Compound 2. One kind, or a plurality of kinds of the compound(s) may be used.

When Compound 1 or Compound 2 is used alone, either Compound 1 (mainly, 4-methylcholest-7-en-3-ol, 4-methylergost-7-en-3-ol, or 4-methylstigmast-7-en-3-ol) or Compound 2 (mainly, 9,19-cyclolanostan-3-ol or 24-methylene-9,19-cyclolanostan-3-ol) is preferable.

Among them, 4-methylcholest-7-en-3-ol is particularly preferable as Compound 1, and 9,19-cyclolanostan-3-ol is particularly preferable as Compound 2, from a view point of physical properties such as solubility which are considered when used as an active ingredient of the drug.

Further, when Compound 1 and Compound 2 are compared, Compound 1 (mainly, 4-methylcholest-7-en-3-ol, 4-methylergost-7-en-3-ol or 4-methylstigmast-7-en-3-ol) is more preferable.

Further, for each of Compound 1 or Compound 2, one kind of a compound may be used, or a plurality of compounds may be used by mixing them.

The inhibitor of the present invention contains, as an active ingredient, preferably a compound selected from the group consisting of 4-methylcholest-7-en-3-ol, 4-methylergost-7-en-3-ol, 4-methylestigmast-7-en-3-ol, 9,19-cyclolanostan-3-ol and 24-methylene-9,19-cyclolanostan-3-ol.

The content of the compound in the inhibitor of the present invention can be appropriately selected depending on symptoms or the like, and the total amount is preferably at least 0.00001% by mass, more preferably at least 0.0001% by mass, further preferably at least 0.0005% by mass, and particularly preferably at least 0.001% by mass. Further, the upper limit of the amount in the inhibitor of the present invention is not particularly limited, but as the total amount, 90% by mass or less, preferably 70% by mass or less and more preferably 50% by mass or less are exemplified.

Further, the inhibitor of the present invention contains, as an active ingredient, a composition comprising a compound selected from the group consisting of Compound 1 and Compound 2. One kind, or a plurality of kinds of the compound(s) may be used.

Such a composition includes, for example, an extract obtained from a plant comprising Compound 1, an extract obtained from a plant comprising Compound 2, an extract obtained from a plant comprising both Compound 1 and Compound 2, described above, as well as a mixture of them.

The inhibitor of the present invention preferably contains both Compound 1 and Compound 2 in combination. One kind, or a plurality of kinds of Compounds 1 and Compounds 2 may be used, respectively. In such an aspect, a composition comprising both Compound 1 and Compound 2 can be used as an active ingredient. The composition includes preferably an extract obtained from a plant comprising both Compound 1 and Compound 2.

When both Compound 1 and Compound 2 are combined, the range of the mass ratio of Compound 1 and Compound 2 includes, for example, the following range:
Compound 1:Compound 2 is preferably 5:1 to 1:5, further preferably 3: 1 to 1:3, and particularly preferably 2: 1 to 1:2.

The inhibitor of the present invention can be used in an aspect of a medicament.

The inhibitor of the present invention can be orally or parenterally administered to a mammal including a human.

The inhibitor of the present invention is effective for preventing or improving symptoms caused by degradation of an extracellular matrix caused by activation of MMP.

In the present invention, the symptom or the disease caused by degradation of an extracellular matrix includes arthritis, osteoarthrosis, rheumatoid arthritis, autoimmune arthritis, periodontal disease, allopatric angiogenesis, infiltration and metastasis of cancers, endometriosis, ulceration, bone disease, vascular reocclusion, vascular restenosis, HIV infectious disease, diabetic complication, arteriosclerosis, aneurysm disease, acute dissecting aortic aneurysm, atherosclerotic plaque dissection, cardiac infarct, cardiac failure, tissue ulcer, wound, skin disease, vesication in bullosis, dyskeratosis, skin disorder such as dermatitis due to ultraviolet ray or the like, skin aging such as epidermal hyperplasia, wrinkle formation, darkness of skin and the like, reduction in skin elasticity, destruction of skin basement membrane, neoplastic angiogenesis, macular degeneration due to age, fibrosis, inflammatory disease due to migrating inflammatory cell, inflammatory bowel disease, corneal ulcer, proteinuria, bullosis, epidermolysis bullosa dystrophica, symptom leading to inflammatory response, osteopenia due to MMP activity, osteoporosis, jaw arthritis, nervous demyelinating disease, regression cartilage loss following tumor metastasis or traumatic joint damage, coronary arterial thrombosis derived from dissection of plaque with atherosclerosis, conception control, lung emphysema, or chronic obstructive lung disease, bedsore, delay of postoperative wound healing term, chronic wound, skin inflammatory of an obese subject, and structural change in dermis.

The inhibitor of the present invention is effective for preventing or improving one or a plurality of symptoms among the above symptoms.

The inhibitor of the present invention is effective for preventing or improving, particularly, symptoms caused by the activity of any MMP selected from the group consisting of MMP-2 and MMP-9 being MMP belonging to a gelatinase group, MMP-12 being a metalloelastase, and MMP-13 belonging to a collagenase group.

For example, the symptom caused by the activity of MMP-2 includes, particularly, infiltration and metastasis of cancers, endometriosis, inflammatory bowel disease, destruction of epidermal basement membrane, bedsore and the like.

The symptom caused by the activity of MMP-9 includes, particularly, epidermal hyperplasia and wrinkle formation of skin, rheumatoid arthritis, acute aortic dissection, bullosis and the like.

The symptom caused by the activity of MMP-12 includes, particularly, arthritis, lung emphysema, aneurysm, arteriosclerosis, chronic obstructive lung disease and the like.

Additionally, the symptom caused by the activity of MMP-13 includes, particularly, osteoarthritis, osteoporosis, periodontitis, multiple sclerosis, gingivitis, corneal epidermal ulcer, bedsore, gastric ulcer, atherosclerosis, vascular restenosis or ischemic cardiac failure after arteriosclerotic obstruction treatment, and the like.

The inhibitor of the present invention is particularly useful for improving or preventing an inflammatory disease. The inflammatory disease includes, for example, gingivitis or periodontitis, tissue ulcer, dermatitis, arthritis, inflammatory bowel disease and the like.

The tissue ulcer includes corneal ulcer, epidermal ulcer, gastric ulcer, ulcerative colitis and the like.

Alternatively, the inhibitor is also useful for improving or preventing a skin disease. The skin disease includes dermatitis, hyperplasia of skin epidermis, wound, dyskeratosis, bullosis, bedsore, delay of postoperative wound healing term, chronic wound, skin inflammation of an obese subject, structural change in dermis, and the like.

The inhibitor of the present invention is particularly effective for preventing or improving symptoms caused by the activity of MMP-2.

The form of the inhibitor of the present invention is not particularly limited, and can be appropriately selected depending on a usage. Specifically, tablets, pills, powders, liquids, suspensions, emulsions, granules, capsules, syrups, suppositories, injections, ointments, patches, eye drops, nose drops and the like can be exemplified.

The administration term of the inhibitor of the present invention is not particularly limited, and can be appropriately selected depending on a subject disease. Further, it is preferable that the dose is determined depending on a dosage form, dose regimen, age and sex of a patient, other conditions, degree of symptom and the like.

The dose of the inhibitor of the present invention is appropriately selected depending on a dose regimen, age and sex of a patient, a severity of a disease, other conditions and the like. Usually, as the dose in terms of an amount of an active ingredient, a standard is preferably in the range of 0.0001 to 100 mg/day, more preferably 0.001 to 50 mg/day, and particularly preferably 0.01 to 10 mg/day.

The inhibitor of the present invention may contain additives which are generally used in a medicament. The additives include an excipient, a binder, a disintegrating agent, a lubricant, a stabilizer, a flavoring agent, a diluent, a surfactant, a solvent for injection and the like.

The inhibitor of the present invention can be manufactured by blending the above-described compound as an active ingredient in a pharmaceutical carrier. The inhibitor of the present invention can be manufactured, for example, by formulating the compound together with the additives described above into a preparation.

Alternatively, the inhibitor of the present invention can also be manufactured by formulating a compound obtained by extraction using hot water or various solvents, supercritical extraction, or subcritical extraction, using the known plant containing the compound or the like as a raw material, together with the additives into preparations.

Particularly, the inhibitor of the present invention comprising Compound 1 and Compound 2 in the specific range of the mass ratio can be manufactured by mixing respective compounds in the above-mentioned range of the mass ratio. Alternatively, such a medicament can also be manufactured by a method of extraction, supercritical extraction, subcritical extraction or the like using various solvents, using the known plant containing a mixture containing Compound 1 and Compound 2 or the like as a raw material.

The inhibitor of the present invention can be obtained, for example, from plants of family Liliaceae, family Leguminosae, family Gramineae, family Solanaceae, and family Musaceae family.

In the inhibitor of the present invention, the above-described compound functions as an active ingredient, and the inhibitor has an action of preventing or improving symptoms due to degradation of an extracellular matrix by the activity of MMP. The inhibitor is effective, particularly, in preventing or improving symptoms due to degradation of an extracellular matrix by the activity of any MMP selected from the group consisting of MMP-2, MMP-9, MMP-12 and MMP-13.

The inhibitor of the present invention is effective, particularly, in preventing or improving symptoms due to degradation of an extracellular matrix by the activity of MMP-2.

The inhibitor of the present invention can also be processed into food or drink by mixing it with raw materials which can be used in food or drink. In the present invention, "food or drink" includes feed which is ingested by animals other than a human, in addition to food or drink which is ingested by a human.

When food or drink is manufactured, the amount of the compound in them is, as the total amount, preferably at least 0.00001% by mass, more preferably at least 0.0001% by mass, further preferably at least 0.0005% by mass, and particularly preferably at least 0.001% by mass. Further, the upper limit of the amount in food or drink of the present invention is not particularly limited, but as the total amount, 90% by mass or less, preferably 70% by mass or less, and more preferably 50% by mass or less are exemplified.

Further, the amount of the compound in food or drink can also be made to be a suitable amount for the compound to be ingested in the range of preferably 0.0001 to 100 mg/day, more preferably 0.001 to 50 mg/day, and particularly preferably 0.01 to 10 mg/day, as expressed by the total amount, depending on the form thereof. Accordingly, one of preferable forms of food or drink of the present invention is food or drink which is used so that the compound is ingested at preferably 0.0001 to 100 mg/day, more preferably 0.001 to 50 mg/day, and particularly preferably 0.01 to 10 mg/day, as expressed by the total amount.

The food or drink is preferably a health functional food or drink. The "health functional food or drink" means a food or drink which directly or indirectly indicates the effect of preventing a disease, or the effect of reducing an onset risk of a disease, and a food or drink which was notified at Consumer Affairs Agency as indicating the functionality on a merchandise package based on scientific basis under business operator's responsibility. Examples thereof include foods or drinks which are currently sold as a food for specified health use, a food with function claims, a health supplement or the like in Japan.

The form of food or drink is not particularly limited, but drinks such as a soft drink, a carbonated drink, a nutritional drink, a fruit juice drink, a lactic acid bacteria beverage and the like (including concentrated original liquid and powder for preparation of these drinks) are particularly preferable from a view point that the compound is effectively ingested.

Further, it is preferable that the form of functional food or drink is a granule, tablet or liquid supplement, from a view point that a person who ingests it can easily grasp the ingestion amount of an active ingredient.

Further, it is preferable that such a functional food or drink is in a form with an indication of use of "for inhibiting production of a matrix metalloproteinase" or "for protecting an extracellular matrix" attached thereto. That is, it is preferable that the food or drink of the present invention is sold as food or drink for preventing or improving symptoms caused by the activity of a matrix metalloproteinase, which comprises a compound selected from the group consisting of Compound 1 and Compound 2 as an active ingredient, for example, with an indication of use of "for protecting an extracellular matrix" attached thereto.

The "indication" includes all indications having the function of informing consumers of the use. That is, indications which can evoke or analogize the use all correspond to the "indication", irrespective of an object of an indication, the content of an indication, a subject to be indicated, a medium and the like with which an indication is performed.

Further, the "with an indication attached thereto" refers to that an indication act of making consumers recognize the indication associated with food or drink (products) exists.

It is preferable that an indication act is such that consumers can directly recognize the use. Specifically, an act of describing the use on merchandise related to the food or drink of the present invention or a package of merchandise, and an act of describing the use on advertisement regarding merchandise, a price list or transaction documents (including those provided by electromagnetic method) are exemplified.

On the other hand, it is preferable that the content to be indicated (indication content) is an indication approved by administration or the like (for example, indication which received approval based on various institutions provided by administration, and is performed in an aspect based on such approval).

For example, indications of a health food, functional food or drink, an enteral nutritive food, a food for special dietary uses, a food with health claims, a special health food, a food with nutrient function claims, a food with function claims, a quasi-drug and the like can be exemplified. Particularly, indication approved by Consumer Affairs Agency, for example, indications approved by special health food institution, or institutions similar thereto can be exemplified. As an example of the latter, an indication as a special health food, an indication as a conditional special health food, an indication to the effect that a structure or the function of a body is influenced, a disease risk reducing indication and the like can be exemplified, more particularly, an indication as a special health food provided in Health Promotion Act, Enforcement Regulation (2003 April 30 Japanese Ordinance of the Ministry of Health, Labour and Welfare No. 86) (particularly, indication of use of health), and indications similar thereto can be mentioned as a typical example.

The term expressing the use is not limited to the phrase of "for inhibiting production of a matrix metalloproteinase" and "for protecting an extracellular matrix", and it goes without saying that other phrases are included in the scope of the present invention as far as they are phrases expressing the action or the effect of preventing or improving symptoms caused by degradation of an extracellular matrix.

Further, it is also preferable that the food or drink of the present invention includes an indication of the active ingredient, and further, an indication showing the relevancy between the use and the active ingredient, in addition to the indication of use.

The food or drink can be manufactured by blending a compound selected from Compound 1 and Compound 2 as an active ingredient. The food or drink of the present invention can be manufactured, for example, by mixing the compound into food or drink raw materials, followed by processing.

Alternatively, the food or drink can also be manufactured by processing an extract obtained by extraction using hot water or various solvents, supercritical extraction, or subcritical extraction using the known plant containing the compound or the like as a raw material, together with food or drink raw materials.

Further, when a form of the food or drink is made to be a granular, tablet-shaped or liquid supplement, it is also preferable that the compound being an active ingredient together with, for example, saccharides such as lactulose, maltitol, and lactitol, and other saccharides, for example, dextrin, starch and the like; proteins such as gelatin, soybean protein, and corn protein; amino acids such as alanine, glutamine, and isoleucine; polysaccharides such as cellulose and gum arabic; fats or oils such as soybean oil, and medium chain fatty acid triglyceride is formulated into a preparation.

### EXAMPLES

The present invention will be illustrated in more detail below by way of Examples, but the present invention is not limited to the following Examples.

### [Production Example 1]

### (Production of Lophenol Compound (Compound 1))

Mesophyll of aloe vera (transparent gel portion) (100 kg) was liquefied using a homogenizer, and 100 L of an ethyl acetate/butanol (3:1) mixed liquid was added thereto, followed by stirring. After allowing to stand overnight, the ethyl acetate/butanol mixed liquid and an aqueous layer were separated to recover the ethyl acetate/butanol mixed liquid. This ethyl acetate/butanol mixed liquid was concentrated under reduced pressure. The mass of the recovered ethyl acetate/butanol mixed liquid extract was 13.5 g.

A solution obtained by dissolving 13 g of the extract in 1 ml of a chloroform/methanol (1:1) mixed liquid was passed through a column filled with 400 g of Silica Gel 60 (manufactured by Merck & Co., Inc.), the extract was adsorbed thereon, then, eluted by a stepwise gradient method of stepwisely increasing the methanol concentration using a chloroform/methanol mixed liquid (each mixing ratio of chloroform: methanol=100:1, 25:1, 10:1, 5:1 and 1:1), and the eluted liquid was fractionated for every mixed ratio of the mixed liquid. It was confirmed by normal phase and reverse phase thin layer chromatography (manufactured by Merck & Co., Inc., Silica Gel 60F254 and RP-18F2543) that the lophenol compound of the present invention exists in a fraction which had been eluted at chloroform: methanol=25:1, among these fractions.

The solvent of this fraction was removed, then, the residue was dissolved in 1 ml of a chloroform/methanol (1:1) mixed liquid, passed through a column filled with 100 g of Silica Gel 60, adsorbed onto a column, then, eluted with 1100 ml of a hexane/ethyl acetate (4:1) mixed liquid. Eluted fractions were collected by 300 ml (Fraction A), 300 ml (Fraction B), and 500 ml (Fraction C) in this order.

It was confirmed by normal phase and reverse phase thin layer chromatography that the lophenol compound being Compound 1 of the present invention was concentrated in Fraction A, and further separated with a chloroform/hexane (85:15) mixed liquid using HPLC equipped with COSMOSIL C18 (manufactured by Nacalai Tesque, Inc.) to obtain 4-methylcholest-7-en-3-ol, 4-methylergost-7-en-3-ol, and 4-methylstigmast-7-en-3-ol at 1.3 mg, 1.2 mg, and 1 mg, respectively. The structure of each compound was confirmed by mass spectrometry(MS) and NMR.

### [Production Example 2]

### (Production of Cyclolanostane Compound (Compound 2))

To 8.0 g of γ-oryzanol (manufactured by Oryza Oil & Fat Chemical Co., Ltd.) were added 250 ml of distilled water, 50 g of sodium hydroxide, 150 ml of isopropanol, 150 ml of ethanol, and 150 ml of methanol, and heating refluxing was performed for 2 hours using a mantle heater. After the reaction, the reaction liquid was added to 1300 ml of water, and the produced white precipitate was suction-filtered to obtain a solid. In order to wash the remaining alkali, the resulting residue was suspended in 1000 ml of water, and suction filtration was performed again. This operation was repeated two times, and the final residue was lyophilized under reduced pressure to obtain 5.91 g of an oryzanol hydrolysate. The hydrolysate was purified by HPLC to obtain 2435 mg of cycloartenol, and 1543 mg of 24-methylene-9,19-cyclolanostan-3-ol (Compound 2).

Then, using the resulting cycloartenol, 9,19-cyclolanostan-3-ol (Compound 2) was synthesized.

302 mg of cycloartenol, 150 ml of isopropanol, and 1.0 g of a powdery 5% palladium-carrying carbon catalyst were charged, these were sealed in an autoclave, the interior thereof was replaced with a nitrogen gas, and a hydrogen gas was introduced while applying a pressure of 3 kg/cm². This was heated while stirring, and at the time point at which a temperature became 50°C, a pressure of hydrogen was adjusted at 5 kg/cm², and a reaction was performed for 6 hours while retaining a pressure by supplementing absorbed hydrogen. The reaction liquid was filtered to remove the catalyst, concentrated and purified by silica gel column chromatography (developing solvent: chloroform 100%) to obtain 275 mg of 9,19-cyclolanostan-3-ol.

### [Production Example 3]

### (Preparation of sample with mixture of lophenol compound and cyclolanostane compound added thereto)

Using 4-methylcholest-7-en-3-ol, 4-methylergost-7-en-3-ol and 4-methylstigmast-7-en-3-ol obtained in Production Example 1, and 9,19-cyclolanostan-3-ol and 24-methylene-9,19-cyclolanostan-3-ol obtained in Production Example 2, a mixture in which the mass ratio of the lophenol compound and the cyclolanostane compound became lophenol compound (Compound 1):cyclolanostane compound (Compound 2) = 1:1 was obtained.

Using carboxymethylcellulose (CMC: produced by DKS Co., Ltd.), the mixture of the lophenol compound and the cyclolanostane compound was dispersed to prepare a 10000-fold diluted powder. The powder was added to an AIN93G feed at 2% to prepare a test sample.

That is, a test sample comprising Compound 1 (lophenol compound) and Compound 2 (cyclolanostane compound) at a total amount of 0.00002% by mass was produced.

### [Example 1]

In the present Example, an influence of a composition comprising Compound 1 and Compound 2 on change in MMP expression, for an increase in the amount of MMP expression by ultraviolet irradiation, was analyzed.

### (1) Preparation of Sample

In the present Example, the test sample prepared in the Production Example 3 was used. In addition, as a control sample (control), an animal feed AIN93G was used.

### (2) Test Method

Using 8-week old hairless mice (HR-1 mice), one group consisting of 6 animals, the animals were reared for a total of 8 weeks, while the control sample or the test sample was administered to a total of two groups, respectively.

From two weeks after start of rearing, hairless mice were placed into an ultraviolet irradiation device, and irradiated with UvB at a frequency of three times/week. The irradiation dose per one time was initiated at 48 mJ/cm², increased to 200 mJ/cm² stepwisely, and the final total irradiation dose was a total of 2.3 J/cm². At six weeks from start of UVB irradiation, hairless mice of each group were dissected, and a dorsal skin tissue was collected to prepare an extracellular matrix tissue section. The tissue section was observed using an optical microscope (Olympus), and fragmentation and orientation of an extracellular matrix (elastic fiber) of a dermic layer were confirmed. Further, also concerning UVB-non-irradiated mice which had been reared with the control sample without UVB irradiation, the tissue section was similarly observed.

### (3) Test Result

An optical micrograph is shown in Fig. 1. As is clear from Fig. 1, in the control sample group, remarkable fragmentation of elastic fibers of the dermic layer was observed, but in the test sample group, fragmentation of elastic fibers of the dermic layer was suppressed. It was found out by this that, in the state where elastic fibers of the dermic layer are fragmented by ultraviolet irradiation, fragmentation of elastic fibers of the dermic layer can be suppressed by ingesting a composition comprising Compound 1 and Compound 2 (suppression of fragmentation of extracellular matrix of skin tissue).

### [Example 2]

In the present test, the effect of inhibiting production of MMP-2 and MMP-9 by a composition comprising Compound 1 and Compound 2 was confirmed.

### (1) Test Method

Eight week-old female hairless mice purchased from Japan SLC, Inc. were divided into a total of 3 groups, one group consisting of 6 animals, and the animals were reared for a total of 8 weeks while a negative control sample (AIN93G Oriental Yeast Co. , Ltd.), a collagen sample obtained by adding 0.6% by mass of collagen to an animal feed AIN93G, as a positive control sample, and a test sample was administered to respective groups. Further, separately, a negative control sample was administered, and one group of a UVB-non-control group to which an ultraviolet ray is not irradiated was set, and was used as a comparative subject.

From two weeks after start of rearing, hairless mice were placed into an ultraviolet irradiation device, and were irradiated with an ultraviolet ray at a frequency of three times/week. The irradiation dose per one time was initiated from 48 mJ/cm², and increased to 200 mJ/cm² stepwisely, and the final irradiation dose was a total of 2.3 J/cm².

At 6 weeks from start of UVB irradiation, a dorsal skin tissue was collected, frozen with liquid nitrogen, and ruptured with Multi Beads Shocker (Yasui Kikai Corporation), and proteins were extracted using CelLytic MT (Sigma-aldrich). The expression amount of MMP-2 and MMP-9 proteins in the extracted proteins was detected using MMP-2 Detection Kit (RayBio), and MMP-9 Detection Kit (R & D Systems).

### (2) Test Result

Results are shown in Table 1 and Table 2. Numerical values related to MMP proteins expression in Table are actually measured values which were measured by the ELISA method based on the Detection Kit. Further, the p value in Table indicates a significance probability by the Dunnett method. As is clear from Table, in the control test group which was irradiated with UVB, the expression amount of MMP-2 (Table 1), and that of MMP-9 (Table 2) were both greater as compared with the UVB-non-irradiated group. On the other hand, in the test sample group, the expression amount thereof was remarkably suppressed as compared with the control group. Meanwhile, in the collagen sample group being a positive control, the significant effect of suppressing the increase in expression of MMP-2 and MMP-9 was not recognized.
[Table 1]

**(Table 1)**

| UVB irradiation/sample | MMP-2 protein expression (ng/µg) | *p* value |
|---|---|---|
| No UVB irradiation/negative control sample (UVB-non-irradiated group) | 3.6 ± 0.3 | 0.04* |
| UVB irradiation/negative control sample (control sample group) | 5.0 ± 0.4 | - |
| UVB irradiation/test sample | 3.8 ± 0.3 | 0.05* |
| (test sample group) | | |
| UVB irradiation/collagen-containing sample (positive control sample group) | 6.1 ± 0.8 | 0.31 |

[Table 2]

**(Table 2)**

| Ultraviolet stress/sample | MMP-9 protein expression (ng/mg) | *p* value |
|---|---|---|
| No UVB irradiation/negative control sample (UVB-non-irradiated group) | 4.0 ± 1.0 | 0.05* |
| UVB irradiation/negative control sample (control sample group) | 9.0 ± 1.8 | - |
| UVB irradiation/test sample (test sample group) | 3.7 ± 0.6 | 0.02* |
| UVB irradiation/collagen-containing sample (positive control sample group) | 6.8 ± 1.6 | 0.39 |

Further, also concerning a test sample comprising Compound 1 alone at 0.00002% by mass, and a test sample comprising Compound 2 alone at 0. 00002% by mass, the effect was similarly confirmed, and as a result, both test samples showed the same inhibitory effect as that of the above-described test sample, on each of MMP-2 and MMP-9.

Thereby, it was made clear that Compound 1 or Compound 2 each independently shows the inhibitory action on MMP-2 and MMP-9.

Further, in also concerning a test sample comprising Compound 1 and Compound 2 at a total amount of 0.00001% by mass, a test sample comprising Compound 1 alone at 0.00001% by mass, and a test sample comprising Compound 2 alone at 0.00001% by mass, in Production Example 3, the effect was similarly confirmed, and as a result, all test samples showed the same inhibitory effect as that of the above-described test sample, on each of MMP-2 and MMP-9.

### [Example 3]

In the present test, the effect of inhibiting production of MMP-12 and MMP-13 by a composition comprising Compound 1 and Compound 2 was confirmed.

### (1) Test Method

Eight week-old female hairless mice purchased from Japan SLC, Inc. were divided into a total of two groups, one group consisting of 6 animals, and the animals are reared for a total of 8 weeks while a negative control sample (AIN93G, Oriental Yeast Co. , Ltd.), and a test sample were administered to each group. Further, separately, one group of a UVB-non-control group to which a negative control sample was administered and an ultraviolet ray is not irradiated was set, and used as a comparative subject.

From two weeks from start of rearing, hairless mice were placed into an ultraviolet irradiation device, and were irradiated with an ultraviolet ray at a frequency of three times/week. The irradiation dose per one time was initiated at 50 mJ/cm², and increased to 200 mJ/cm² stepwisely, and a final irradiation amount was a total of 3.1 J/cm².

At 6 weeks from start of UVB irradiation, the animals were dissected, a dorsal skin tissue was collected, frozen with liquid nitrogen, and ruptured with Multi Beads Shocker (Yasui Kikai Corporation), and proteins were extracted using CelLytic MT (Sigma-aldrich). The expression amount of MMP-12 and MMP-13 proteins in the extract proteins was detected using MMP-12 Detection Kit (Cloud-Clone) and MMP-13 Detection Kit (Cloud-Clone).

### (2) Test Result

Results are shown in Table 3 and Table 4. Numerical values related to expression of MMP proteins in Table are actually measured values which were measured by the ELISA method based on the Detection Kit. Further, the p value in Table indicates a significance probability by the Dunnett method. As a result, in the negative control sample group to which UVB was irradiated, the expression amount of MMP-12 (Table 3) and that of MMP-13 (Table 4) were both remarkably increased relative to the UVB-non-irradiated group, as is clear from Table. On the other hand, in the test sample group, the expression amount was remarkably suppressed as compared with the control sample, particularly, in MMP-12.

Further, also regarding a test sample comprising Compound 1 alone at 0.00002% by mass, and a test sample comprising Compound 2 alone at 0.00002% by mass, the effect was similarly confirmed, and as a result, both test samples showed the same inhibitory effect as that of the above-described test sample, on each of MMP-12 and MMP-13.

Thereby, it was made clear that Compound 1 and Compound 2 each independently shows the inhibitory action on MMP-12 and MMP-13.

Further, also concerning a test sample comprising Compound 1 and Compound 2 at a total amount of 0.00001% by mass, a test sample comprising Compound 1 alone at 0.00001% by mass, and a test sample comprising Compound 2 alone at 0.00001% by mass, in Production Example 3, the effect was similarly confirmed, and as a result, all test samples showed the same inhibitory effect as that of the above-described test sample, on each of MMP-12 and MMP-13.
[Table 3]

**(Table 3)**

| Ultraviolet stress/sample | MMP-12 protein expression (ng/mg) | *p* value |
|---|---|---|
| No UVB irradiation/negative control sample (UVB-non-irradiated group) | 20.4 ± 1.7 | 0.05* |
| UVB irradiation/negative control sample (control sample group) | 38.8 ± 7.1 | - |
| UVB irradiation/test sample (test sample group) | 21.2 ± 2.8 | 0.06 |

[Table 4]

**(Table 4)**

| Ultraviolet stress/sample | MMP-13 protein expression (ng/mg) | *p* value |
|---|---|---|
| No UVB irradiation/negative control sample (UVB-non-irradiated group) | 2.0 ± 0.2 | 0.02* |
| UVB irradiation/negative control sample (control sample group) | 2.9 ± 0.5 | - |
| UVB irradiation/test sample (test sample group) | 2.2 ± 0.2 | 0.04* |

As shown in Examples 2 and 3 , it was found out that Compound 1 and Compound 2, alone or in combination thereof, remarkably show the inhibitory action on MMP-2 , MMP-9 , MMP-12 , and MMP-13. Particularly, it was surprising that Compound 1 and Compound 2 show the inhibitory action on all of MMP-2 and MMP-9 belonging to a gelatinase group, MMP-12 being a metalloelastase, as well as MMP-13 being collagenase. Particularly, the inhibitory action on MMP-2 can be said to be remarkable among components having relatively high safety which have previously been known as having the MMP inhibitory action. Since Compound 1 and Compound 2 have been confirmed in safety on a living body, they are extremely useful for improving symptoms caused by production of MMP.

### [Comparative Example]

In the present test, the effect of inhibiting production of MMP-3 by a composition comprising Compound 1 and Compound 2, on MMP-3 different from MMPs which were confirmed in Examples 2 and 3, was confirmed.

### (1) Test Method

Eight week-old female hairless mice purchased from Japan SLC, Inc. were divided into a total of 2 groups, one group consisting of 6 animals, and the animals were reared for a total of 8 weeks, while a negative control sample (AIN93G Oriental Yeast Co., Ltd.) and a test sample were administered to each group. Further, separately, one group of a UVB-non-control group to which a negative control sample is administered and an ultraviolet ray is not irradiated was set, and used as a comparative subject.

From two weeks after start of rearing, hairless mice were placed into an ultraviolet irradiation device, and irradiated with an ultraviolet ray at a frequency of three times/week. The irradiation dose per one time was initiated at 50 mJ/cm², and increased to 200 mJ/cm² stepwisely, and a final irradiation amount was a total of 3.1 J/cm².

At 6 weeks from start of UVB irradiation, the animals were dissected, a dorsal skin tissue was collected, frozen with liquid nitrogen and ruptured with Multi Beads Shocker (Yasui Kikai Corporation) and proteins were extracted using CelLytic MT (Sigma-aldrich). The expression amount of MMP-3 protein in the extracted proteins was detected using MMP-3 Detection Kit (BioSource).

### (2) Test Result

Results are shown in Table 5. Numerical values related to MMP protein expression in Table are actually measured values which were measured by the ELISA method based on the Detection Kit. Further, the p value in Table indicates a significance probability by the Dunnett method. As a result, the effect of suppressing MMP-3 expression by the test sample was not observed, for MMP-3 (Table 5), as is clear from Table.
[Table 5]

**(Table 5)**

| Ultraviolet stress/sample | MMP-3 protein expression (ng/mg) | *p* value |
|---|---|---|
| No UVB irradiation/negative control sample (UVB-non-irradiated group) | 6.8 ± 0.2 | 0.99 |
| UVB irradiation/negative control sample (control sample group) | 6.8 ± 1.2 | - |
| UVB irradiation/test sample (test sample group) | 7.1 ± 0.4 | 0.59 |

### [Example 4]

A medicament having the effect of inhibiting production of a matrix metalloproteinase consisting of the following composition was produced by the following method.

Two percent (2%) by mass of a composition prepared by adding carboxymethylcellulose (CMC: manufactured by DKS Co., Ltd.) to a mixture containing the lophenol compound produced in Production Example 1 and the cyclolanostane compound produced in Production Example 2 at a ratio of lophenol compound:cyclolanostane compound = 2:3 and dispersing the materials, the composition containing 0.001% by mass of the mixture, 2% by mass of a medium chain fatty acid (MCT: manufactured by RIKEN VITAMIN CO., LTD.), 4% by mass of a glycerin fatty acid ester (manufactured by RIKEN VITAMIN CO. , LTD.), 0.5% by mass of saponin (manufactured by MARUZEN PHARMACEUTICALS CO., LTD.), 0.2% by mass of ethanol (manufactured by Japan Alcohol Corporation), 1.3% by mass of maltitol (manufactured by HAYASHIBARA CO., LTD.), 78% by mass of glycerin (manufactured by NOF CORPORATION) and, further, water were added and the materials were mixed so that the total amount was 100% by mass, to produce a syrup-like preparation containing the mixture of the lophenol compound (Compound 1) and the cyclolanostane compound (Compound 2) at a final concentration of 0.00002% by mass.

The inhibitor of the present Example is effective for improving or treating symptoms caused by degradation of an extracellular matrix.

### INDUSTRIAL APPLICABILITY

The present invention can be utilized in preventing, improving or treating symptoms due to matrix metalloproteinase production, and degradation of an extracellular matrix by this.

## Claims

1. A matrix metalloproteinase production inhibitor comprising a compound selected from the group consisting of a lophenol compound and a cyclolanostane compound, as an active ingredient.

2. The matrix metalloproteinase production inhibitor according to claim 1, comprising the compound at a total amount of 0.00001% by mass or more.

3. The matrix metalloproteinase production inhibitor according to claim 1 or 2, wherein the matrix metalloproteinase is any one selected from the group consisting of matrix metalloproteinase-2, matrix metalloproteinase-9, matrix metalloproteinase-12 and matrix metalloproteinase-13.

4. Use of a compound selected from the group consisting of a lophenol compound and a cyclolanostane compound in the manufacture of a matrix metalloproteinase production inhibitor.

5. A compound selected from the group consisting of a lophenol compound and a cyclolanostane compound for use in matrix metalloproteinase production inhibition.

6. A composition comprising a compound selected from the group consisting of a lophenol compound and a cyclolanostane compound at a total amount of 0.00001% by mass or more, for use in matrix metalloproteinase production inhibition.

7. The composition according to claim 6, wherein the composition is food or drink.

8. A method of inhibiting production of a matrix metalloproteinase, comprising administering a compound selected from the group consisting of a lophenol compound and a cyclolanostane compound to a subject in need of inhibition of production of a matrix metalloproteinase.
